# EUROPEAN PATENT APPLICATION

(11) **EP 3 539 518 A1**
(43) Date of publication of application: **18.09.2019**
(21) Application number: 18161344.9
(22) Date of filing: 13.03.2018
(51) Int. Cl.: A61F 13/20, A61F 13/26

(54) **MULTI-SEGMENT TAMPON**

(71) Applicant: Chien, Yuan-Cheng, Kaohsiung City (TW)
(72) Inventor: Chien, Yuan-Cheng, Kaohsiung City (TW)
(74) Representative: Boco IP Oy Ab

(57) **Abstract**

A multi-segment tampon (2, 2', 2") includes an absorbent body (3, 3', 3") and a pull string (4). The absorbent body (3, 3', 3") is configured to be inserted into a vagina (6) for absorbing menstrual blood and includes a plurality of body segments (31, 31', 31") arranged along a longitudinal direction (A). Each body segment (31, 31', 31") defines an insert hole (32). The pull string (4) has one end extending into the insert holes (32) of the body segments (31, 31', 31") in order to string the body segments (31, 31', 31") together along the longitudinal direction (A). Another end of the pull string (4) that is opposite to the one end extends out of the absorbent body (3, 3', 3").

## Description

### 1. Field of the Invention

The present invention relates to a hygiene product, and more particularly to a multi-segment tampon.

### 2. Description of Related Art

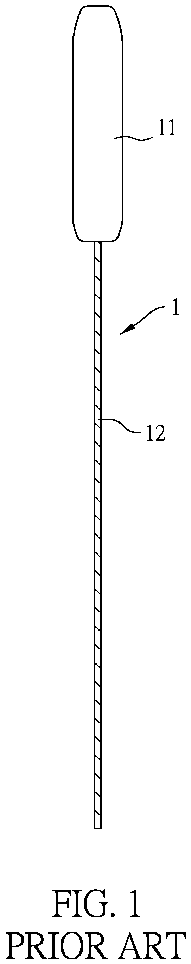

In addition to sanitary napkins, tampons are also commonly used by women. Unlike the sanitary napkins, the tampon is comfortable to use without muggy feeling and will not cause discomfort to the user during walking due to friction. With reference to Fig. 1, a conventional tampon 1 includes an absorbent body 11 having a generally cylindrical shape and a curved front end surface, and a pull string 12 connected to the absorbent body 11. In use, the absorbent body 11 is inserted into the vagina for absorbing menstrual blood to achieve the effect of preventing leakage of the menstrual blood and cleaning. The pull string 12 is pulled to remove the absorbent body 11 from the vagina.

Although using the conventional tampon 1 is less susceptible to the muggy feeling as compared to the sanitary napkins, however, the entire outer circumferential surface of the absorbent body 11 is in contact with the vagina, thus improvement in air permeability thereof is needed. Moreover, although the conventional tampon 1 is less obtrusive to physical activity, the absorbent body 11 is not designed to be fully bendable. Hence, the conventional tampon 1 is inconvenient to use when strenuous physical activity is taken, and there remains room for improvement in comfort to the user.

To overcome the shortcomings, the present invention provides a multi-segment tampon to mitigate or obviate the aforementioned problems.

An objective of the present invention is to provide a multi-segment tampon that has good air permeability.

Another objective of the present invention is to provide a multi-segment tampon that has good conformability to the vagina structure of any user.

Another objective of the present invention is to provide a multi-segment tampon that prevents the leakage of the menstrual blood.

According to the present invention, a multi-segment tampon includes an absorbent body and a pull string. The absorbent body is configured to be inserted into a vagina for absorbing menstrual blood and includes a plurality of body segments arranged along a longitudinal direction. Each of the body segments defines an insert hole. The pull string has one end extending into the insert holes of the body segments in order to string the body segments together along the longitudinal direction. Another end of the pull string that is opposite to the one end extends out of the absorbent body.

Other objectives, advantages and features of the present invention will become more apparent from the following detailed description when taken in conjunction with the accompanying drawings.

### IN THE DRAWINGS:

Fig. 1 is a schematic view of a conventional tampon;
Fig. 2 is a perspective view of a first embodiment of a multi-segment tampon in accordance with the present invention;
Fig. 3 is a sectional view of the first embodiment;
Fig. 4 illustrates the first embodiment in a state of use;
Fig. 5 is a view similar to Fig. 4, but with an auxiliary device being further inserted into the vagina;
Fig. 6 is a view similar to Fig. 5, but with the auxiliary device being removed from the vagina;
Fig. 7 is a sectional view of a second embodiment of a multi-segment tampon in accordance with the present invention;
Fig. 8 is a view similar to Fig. 7, but with a liquid blocking film adhered to a rearmost one of the body segments;
Fig. 9 illustrates the second embodiment in a state of use;
Fig. 10 is a sectional view of an alternative form of the second embodiment;
Fig. 11 is a sectional view of a third embodiment of a multi-segment tampon in accordance with the present invention;
Fig. 12 is a sectional view of a state of the third embodiment after absorbing menstrual blood;
Fig. 13 is a sectional view of a fourth embodiment of a multi-segment tampon in accordance with the present invention; and
Fig. 14 is a sectional view of a state of the fourth embodiment after absorbing menstrual blood.

Before the present invention is described in greater detail with reference to the accompanying embodiment, it should be noted herein that like elements are denoted by the same reference numerals throughout the disclosure of the present invention.

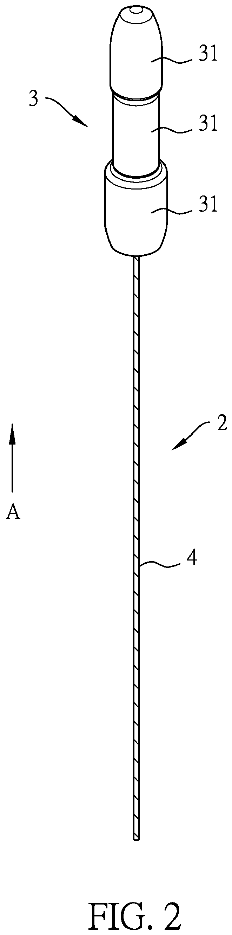

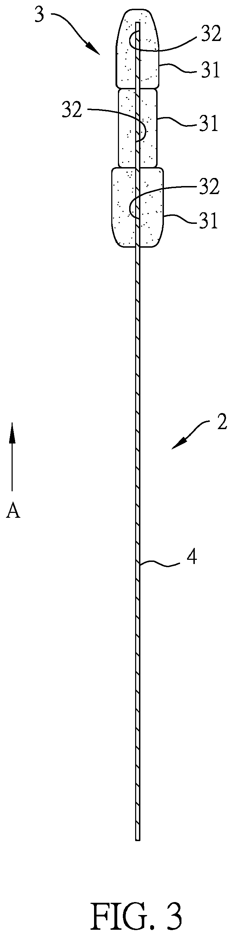

With reference to Figs. 2 and 3, a first embodiment of a multi-segment tampon 2 of the present invention includes an absorbent body 3 and a pull string 4. The absorbent body 3 is configured to be inserted into a vagina 6 for absorbing menstrual blood, and includes a plurality of body segments 31 arranged along a longitudinal direction A. In this embodiment, three body segments 31 are exemplified. Each of the body segments 31 has a columnar shape, and defines an insert hole 32 extending along the length thereof. The pull string 4 has one end extending into the insert holes 32 of the body segments 31 and fixed to an outermost one, that is, a foremost one, of the body segments 31 so as to string the body segments 31 together along the longitudinal direction A, and another end opposite to the one end and extending out of the absorbent body 3.

To take into account the comfort of the user when inserting the tampon 2 into the vagina 6, a leading edge of the foremost one of the body segments 31 and a trailing edge of a rearmost one of the body segments 31 are designed with a curved shape.

Further, the body segments 31 have unequal outer diameters. As shown in Fig. 3, the rearmost body segment 31 has the largest outer diameter, the foremost body segment 31 has the second largest outer diameter, while the outer diameter of the middle body segment 31 is the smallest.

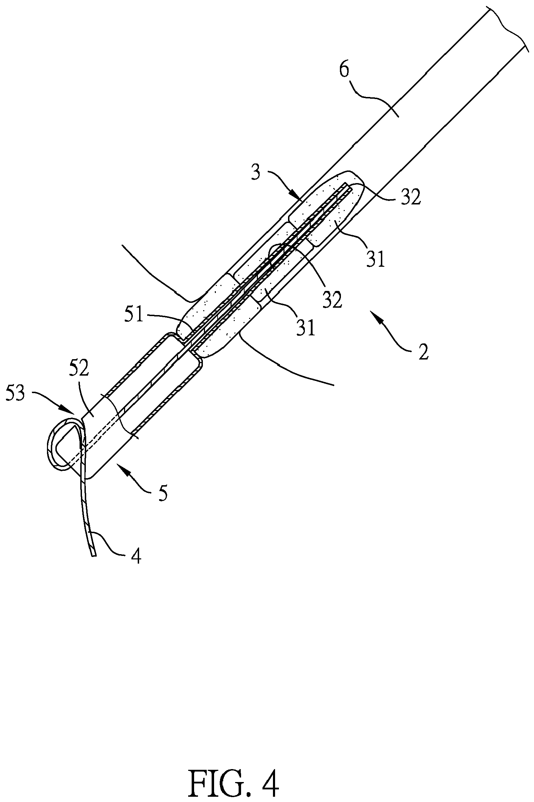

With reference to Figs. 4, 5 and 6, the first embodiment of the multi-segment tampon 2 of the present invention further includes an auxiliary device 5. The auxiliary device 5 is hollow to allow insertion of the pull string 4 therethrough. The auxiliary device 5 includes an insert rod 51 removably inserted into the insert holes 32 of the body segments 31, and a handgrip 52 fixedly connected to a rear end of the insert rod 51. The insert rod 51 has a hollow conical shape that tapers from a rear end to a front end thereof, so that the insert rod 51 can be easily pulled out of the insert holes 32 and will not be subjected to the thermal expansion and contraction of the absorbent body 3. As such, the insert rod 51 will not be stuck in the insert holes 32.

Before insertion of the multi-segment tampon 2 into the vagina 6, the insert rod 51 of the auxiliary device 5 is first inserted into the insert holes 32 of the body segments 31, so that the body segments 31 are disposed coaxially to facilitate the insertion thereof. At the same time, the other end of the pull string 4 passes through the insert rod 51 and the handgrip 52. The handgrip 52 is formed with a notch 53, as shown in Fig. 4. The notch 53 is used for insertion of the other end of the pull string 4 therethrough after extending out of the handgrip 52 for avoiding the absorbent body 3 to be pulled directly when the pull string 4 is accidentally or unintentionally tugged. It should be noted herein that the front end of the insert rod 51 does not extend through the foremost body segment 31, but rather it abuts against an inner side of the foremost body segment 31.

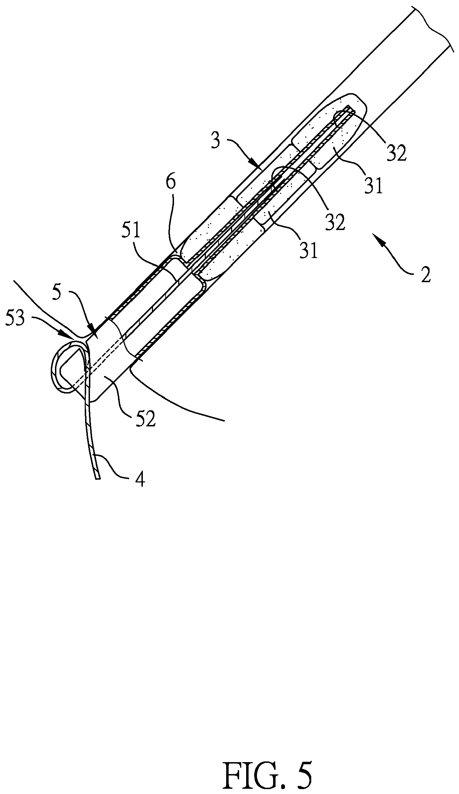

To insert the multi-segment tampon 2 into the vagina 6, a user first grips the handgrip 52, after which the absorbent body 3 together with the insert rod 51 is pushed into the vagina 6, as shown in Fig. 4, and is placed in a proper position, as shown in Fig. 5. Following the insertion of the multi-segment tampon 2 into the proper position in the vagina 6, the handgrip 52 is pulled rearwardly to remove the auxiliary device 5 from the absorbent body 3 and out of the vagina 6, thereby completing the positioning of the multi-segment tampon 2.

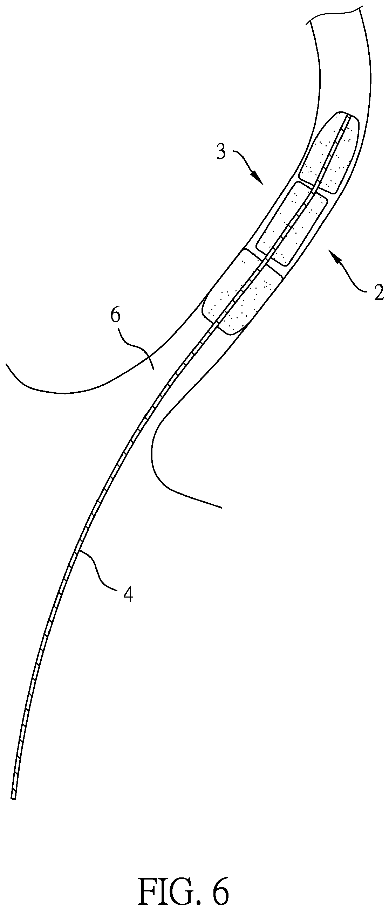

Since the outer diameters of the body segments 31 are unequal so that the body segment 31 with the smaller outer diameter will not contact the vagina 6, and since gaps exist between each two adjacent ones of the body segments 31 after the removal of the auxiliary device 5 from the vagina 6, as shown in Fig. 6, the multi-segment tampon 2 of the first embodiment has a better air permeability compared to the conventional tampon 1.

Further, the absorbent body 3 is designed with multiple body segments 31 that are able to bend and deform relative to each other so as to allow a large movement of the user, so that each body segment 31 can correspondingly fit to an inner wall of the vagina 6, i.e. the absorbent body 3 has good conformability to the structure of vagina 6, thereby reducing the feeling of the presence of a foreign matter in the vagina 6, and thereby improving the user's comfort.

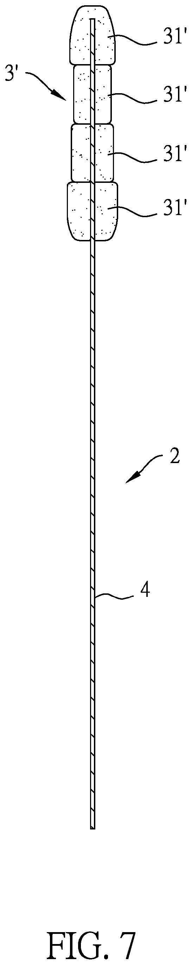

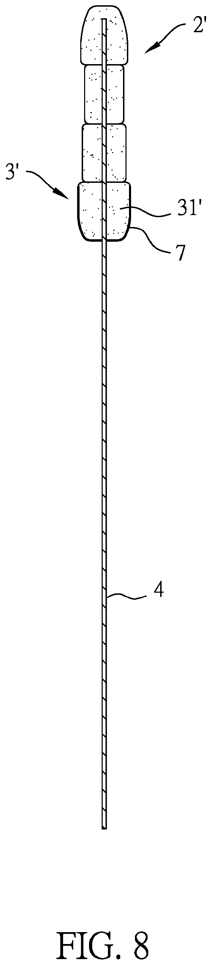

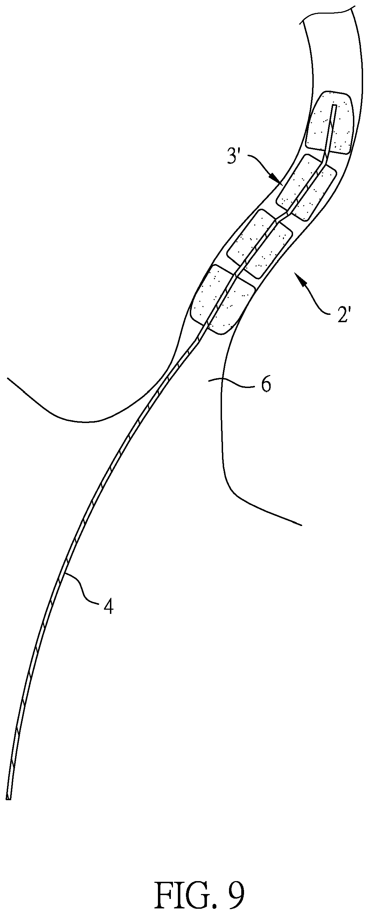

In addition, since gaps exist between each two adjacent ones of the body segments 31 in the vagina 6, as shown in Fig. 6, the multiple body segments 31 can expand well after absorbing the menstrual blood, and therefore the multiple body segments 31 fit with the inner wall of the vagina 6 and increase the area of contact therewith to prevent leakage of the menstrual blood. With reference to Figs. 7, 8 and 9, a second embodiment of the multi-segment tampon 2' of the present invention is shown to be generally identical to the first embodiment, and differs in that, in the second embodiment, the number of the body segments 31' of the absorbent body 3' is four. Depending on the user's requirement, it is possible to disassemble or add a specific number of the body segments 31' of the absorbent body 3', and alter the outer diameters of the body segments 31'. For example, the outer diameters of several of the body segments 31' may be the same, while the outer diameters of the other body segments 31' may differ.

The multi-segment tampon 2' of the second embodiment further includes a liquid blocking film 7 adhered to the rearmost one of the body segments 31'. The liquid blocking film 7 enhances the effect of blocking liquid to prevent leakage of the menstrual blood from the absorbent body 3', so that users with large amount of menstrual blood need not use the sanitary napkins to absorb the additional menstrual blood, thereby increasing the convenience of using the multi-segment tampon 2'.

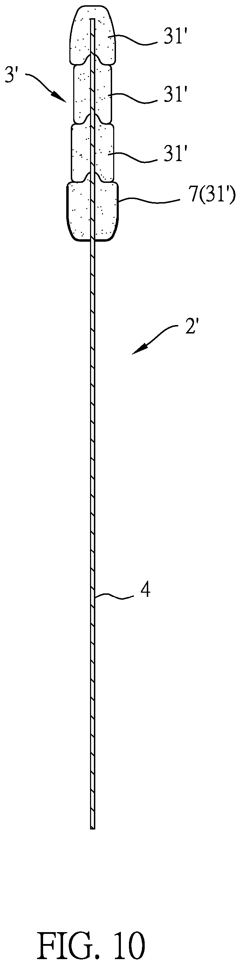

Fig. 10 illustrates an alternative form of the body segments 31' of the absorbent body 3' of the second embodiment, but is not limited thereto.

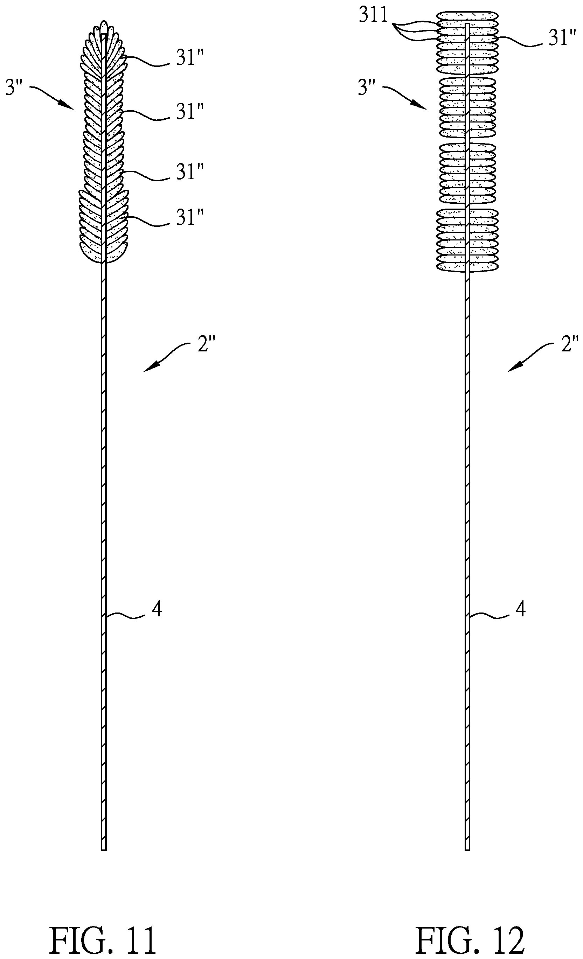

With reference to Figs. 11 and 12, a third embodiment of a multi-segment tampon 2" of the present invention is shown to be generally identical to the first embodiment, and differs in that, in the third embodiment, each body segment 31" of the absorbent body 3" includes a plurality of cotton pads 311 stacked one upon another. Each of the cotton pads 311 of the body segments 31" has a substantially V-shape, and two opposite sides extending obliquely and upwardly away from each other and cooperatively defining an angle of 130 degrees therebetween.

As shown in Fig. 12, after the cotton pads 311 of the body segments 31" absorb the menstrual blood, the cotton pads 311 will expand and move downward so that the body segments 31" can correspondingly contact the inner wall of the vagina 6.

Further, gaps between each two adjacent ones of the body segments 31" are also present in this embodiment, so that air permeability of the multi-segment tampon 2" of this embodiment can be ensured.

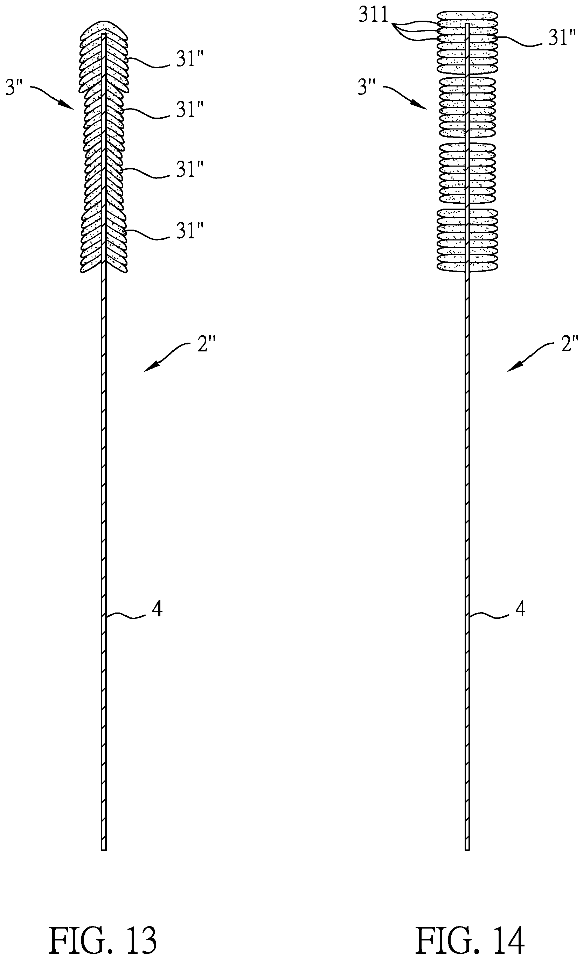

With reference to Figs. 13 and 14, a fourth embodiment of the multi-segment tampon 2" of the present invention is shown to be generally identical to the third embodiment, and differs in that, in the fourth embodiment, each of the cotton pads 311 of the body segments 31" of the absorbent body 3" has a substantially inverted V-shape. As shown in Fig. 14, after the cotton pads 311 of the body segments 31" absorb the menstrual blood, the cotton pads 311 will expand and move upward, so that the body segments 31" can correspondingly contact the inner wall of the vagina 6.

In summary, because the multiple body segments 31, 31', 31" of the absorbent body 3, 3', 3" of the multi-segment tampon 2, 2', 2" of the present invention have unequal outer diameters but creating gaps in between each two adjacent ones of the body segments 31, 31', 31", air permeability is enhanced and the muggy feeling is reduced.

Further, because the body segments 31, 31', 31" are able to bend and deform relative to each other and can correspondingly fit and contact the inner wall of the vagina 6, the multi-segment tampon 2, 2', 2" of the present invention has good conformability in the structure of the vagina 6, so as to reduce the feeling of intrusion by a foreign element, thus enhancing the comfort of use.

Further, since gaps exist between each two adjacent ones of the body segments 31, 31', 31", the multiple body segments 31, 31', 31" have room to expand well after absorbing the menstrual blood, and therefore the multiple body segments 31, 31', 31" fit with the inner wall of the vagina and increase the area of contact therewith to prevent leakage of the menstrual blood.

In addition, the number of the body segments 31, 31', 31" of the absorbent body 3, 3', 3" can be altered according to the requirement of the user. Hence, the objective of the present invention can indeed be achieved.

In the description above, for the purposes of explanation, numerous specific details have been set forth in order to provide a thorough understanding of the embodiment. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the present invention. It should be further appreciated that in the description, various features are sometimes grouped together in a single embodiment, figure, or description thereof for the purpose of streamlining the present invention and aiding in the understanding of various inventive aspects.

While the disclosure has been described in connection with what are considered the exemplary embodiments, it is understood that this disclosure is not limited to the disclosed embodiments but is intended to cover various arrangements included within the spirit and scope of the broadest interpretation so as to encompass all such modifications and equivalent arrangements.

## Claims

1. A multi-segment tampon (2, 2', 2") comprising an absorbent body (3, 3', 3") configured to be inserted into a vagina (6) for absorbing menstrual blood, and a pull string (4) connected to said absorbent body (3, 3', 3"), **characterized by**:
said absorbent body (3, 3', 3") including a plurality of body segments (31, 31', 31") arranged along a longitudinal direction (A), each of said body segments (31, 31', 31") defining an insert hole (32); and
said pull string (4) having one end extending into said insert holes (32) of said body segments (31, 31', 31") to string said body segments (31, 31', 31") together along the longitudinal direction (A), and another end opposite to said one end and extending out of said absorbent body (3, 3', 3").

2. The multi-segment tampon (2, 2', 2") as claimed in claim 1, wherein said body segments (31, 31', 31") have unequal outer diameters.

3. The multi-segment tampon (2, 2', 2") as claimed in claim 1, wherein said one end of said pull string (4) is fixed to one of two outermost ones of said body segments (31, 31', 31").

4. The multi-segment tampon (2') as claimed in claim 3, wherein a liquid blocking film (7) is adhered to the other one of said two outermost ones of said body segments (31').

5. The multi-segment tampon (2, 2', 2") as claimed in claim 1, wherein an auxiliary device (5) is hollow for insertion of said pull string (4) therethrough and includes an insert rod (51) removably inserted into said insert holes (32) of said body segments (31, 31', 31"), and a handgrip (52) is fixedly connected to a rear end of said insert rod (51).

6. The multi-segment tampon (2, 2', 2") as claimed in claim 5, wherein said insert rod (51) has a hollow conical shape that tapers from a rear end to a front end thereof.

7. The multi-segment tampon (2") as claimed in claim 1, wherein each of said body segments (31") includes a plurality of cotton pads (311) stacked one upon another.

8. The multi-segment tampon (2") as claimed in claim 7, wherein each of said cotton pads (311) has a substantially V-shape.

9. The multi-segment tampon (2") as claimed in claim 7, wherein each of said cotton pads (311) has a substantially inverted V-shape.
